# EUROPEAN PATENT SPECIFICATION

(11) **EP 3 034 512 B1**
(45) Date of publication and mention of the grant of the patent: **06.12.2017**
(21) Application number: 14386034.4
(22) Date of filing: 19.12.2014
(51) Int. Cl.: C07K 14/47

(54) **A process for producing iron (III) casein N-acetyl-aspartylated complexes and use thereof in pharmaceutical compositions**
Verfahren zur Herstellung von Eisen (III)-Casein-N-Acetyl-aspartylierten Komplexen und Verwendung derselben in pharmazeutischen Zusammensetzungen
Procédé de production de complexes fer (III) de la caséine N-acétyl-aspartyl et leur utilisation dans des compositions pharmaceutiques

(43) Date of publication of application: 22.06.2016
(73) Proprietor: Tseti, Ioulia, 145 61 Kifissia Attikis (GR)
(72) Inventor: Tseti, Ioulia, 145 61 Kifissia Attikis (GR)
(74) Representative: Wibbelmann, Jobst

(56) References cited:
- EP-B1- 0 243 322
- WO-A1-2006/021843
- LAZZARI FLAVIO ET AL: "Overview of clinical trials in the treatment of iron deficiency with iron-acetyl-aspartylated casein", CLINICAL DRUG INVESTIGATION, vol. 25, no. 11, 2005, pages 679-689, XP009182971, ISSN: 1173-2563
- BURON ET AL: "On the racemisation of aspartic anhydride during its preparation", TETRAHEDRON ASYMMETRY, PERGAMON PRESS LTD, OXFORD, GB, vol. 18, no. 13, 17 July 2007 (2007-07-17) , pages 1625-1627, XP022181932, ISSN: 0957-4166, DOI: 10.1016/J.TETASY.2007.07.003

## Description

The present invention generally relates to iron (III) protein complexes and to processes for the manufacture thereof. The product obtainable according to the method of the present invention may be safely used for administration to the general population or animals in the therapy of iron deficiency.

### BACKGROUND ART

Iron therapy is necessary in a wide variety of clinical situations including pregnancy, chemotherapy, acute or chronic hemorrhage, hemodialysis, inflammatory bowel disease, inadequate diet, and gastrointestinal surgery.

Oral iron therapy is considerably less expensive than the alternative parenteral iron therapy and also poses lower risks of serious allergic reactions. On this base, in British patent application no 910321 (Olivieur Paul Gaudin), an improved process is described for the preparation of ferrous salts with aspartic acid. These salts could be used as medicines for iron deficiency.

Cremonesi P et al, in 1984 (Arzneimittel-Forschung, 34(9):948-952) reported the first Iron derivatives of modified milk protein. Moreover, it is stated that a product containing a high amount of iron and maintaining all the necessary characteristics of stability and solubility for a drug can be prepared by succinylating the milk proteins, before the reaction with the iron salt. For these derivatives a particular advantage could be achieved. Iron, in the form of oligomeric complex, is tightly bonded to the protein not in chelated structures with basic residues but involving several sites of the protein chain. The solubility of the iron complex is assured by the increased availability of carboxyl groups that follows the succinylation reaction.

US Patent No. 4493829, the same period, describes succinylated protein-iron derivatives with no gastric lesions and improved bio-availability. The same protein (vegetable and animal) modifications are described in European patent no. 0243322, by reaction of the proteins with 3-10 aliphatic dicarboxylic acid anhydrides, introducing carboxylic acid groups to the protein, thus facilitating the complexation of the iron.

Chemical and biological characterization of iron-protein succinylate in rats and dogs are described by Cremonesi P et al. (International Journal of Clinical Pharmacology, therapy, and toxicology, 31(1):40-51). The experiments demonstrated better gastrointestinal tolerability to an iron succinyl casein complex containing 5 % iron.

EP0939083 discloses process for the preparation of a ferro-succinylcasein complex obtained from food-grade casein used for food purposes.

US2001031748 refers to dietary of various metals, iron included, in combination to a dietary ligand administration such as ascorbate, succinate, aspartate and other ligands. The combination of such a dietary can assist to treating gastrointestinal symptoms.

Lazzari et al. reported (Clinical Drug Investigation, 2005; 25(11):679-689) an overview of 16 clinical trials in the treatment of iron deficiency with iron-acetyl-aspartylated casein (Fe-ASP), which proved to be an efficient vehicle for providing iron with high bioavailability. In open clinical trials, highly significant improvements in clinical and haematological parameters were observed after treatment with Fe-ASP in all categories of patients with iron deficiency anaemia. In controlled clinical trials, the changes in clinical and haematological profiles observed with Fe-ASP were virtually identical to those seen with iron protein succinylate (IPS), and Fe-ASP also compared well with parenteral iron gluconate. No safety considerations were raised. Fe-ASP shows high efficacy in iron-deficient anaemia treatment, and it is an extremely well tolerated iron vehicle. Fe-ASP represents a valid alternative to IPS and shows promise as a substitute for parenteral iron therapy in selected clinical situations.

More recent, WO patent No. 2006021843 discloses a process for producing iron succinyl casein and acetyl-aspartate iron casein complexes in which the substance is little exposed to pH and temperature conditions. The process uses acetyl-aspartate anhydride. Moreover, this process is required no use of special pumps (dilacerations pumps).

### SUMMARY OF THE INVENTION

The present invention concerns a process for the preparation of iron (III) complex with N-acetyl-L-aspartylated casein, comprising the steps of (a) reaction of casein with N-acetyl-L-aspartyl chloride, to form N-acetyl-L-aspartylated casein, (b) subsequent reaction of the N-acetyl-L-aspartylated casein with ferric chloride; and (c) obtaining the iron (III) complex with N-acetyl-L-aspartylated casein.

Moreover, the present invention relates to a process for preparing a pharmaceutical composition, comprising an iron (III) complex with N-acetyl-L-aspartylated food-grade casein, said process comprising the process for the preparation of iron (III) complex as set forth in claims 1-5, and combining said iron (III) complex with N-acetyl-L-aspartylated food-grade casein with pharmaceutical excipients, in particular selected from diluents and preservatives.

The complexes of the present invention are stable and show a high stability over time. The products may therefore be used for the therapy of iron deficiency in humans or animals.

### DETAILED DESCRIPTION OF THE INVENTION

The present invention is set forth in greater detail in the attached dependent claims.

Thus, the present invention suggests a new process for producing protein derivatives of iron, namely, a ferric complex of N-acetyl-L-aspartylated casein (hereinafter referred to as "ferro-N-acetyl-L-aspartylated casein" and iron (III) complex with N-acetyl-L-aspartylated casein), preferably obtained from food-grade casein, i.e. casein used for food purposes. By the term "food-grade casein" is meant casein obtained from milk coming from strictly controlled breeding farms. This product presents a level of contamination lower than 1000 CFU/g for bacteria, and lower than 100 CFU/g for moulds, which contamination levels are preferably determined as described in current Eur. Pharm, monographs *2.6.12 for Total viable aerobic count, 2.6.13 for specified microorganisms* and *2.6.14 for Bacterial endotoxins* The use of food-grade casein allows obtaining particularly pure products, as compared to similar complexes obtained starting from usual milk proteins.

The present invention refers to a process for the preparation of complexes of iron (III) casein N-acetyl-L-aspartylated comprising, preferably consisting of, the following steps: (a) N-acetyl-L-aspartylation of casein, preferably food-grade casein, with N-acetyl-L-aspartic acid chloride, prepared *in situ* by chlorination of the N-acetyl-L-aspartic acid with a chlorinating reagent such as thionyl chloride. Although N-acetyl aspartic anhydride is generally a useful starting material for casein modification, it has several disadvantages. For example, it is relative expensive for industrial use and also unstable against humidity on storage. Moreover there is a risk for N-acetyl aspartic anhydride of partial racemization (Tetrahedron: Asymmetry 18 (2007) 1625-1627). That for, the use of N-acetyl-L-aspartic acid in combination with the chlorinating reagent advantages over the use of the N-acetyl aspartic anhydride in the context of the present application. (b) Subsequent reaction of the N-acetyl-L-aspartylated casein with ferric chloride. Step (a) preferably comprises providing dissolved casein.

Preferred embodiments are described in detail as follows, wherein each of stages 1 to 9 can be used alone or in combination with any other stages for defining the present invention, most preferably all stages 1 to 9 are applied as described below:

### Step (a):

In stage 1), dissolving of casein, preferably food-grade, is performed in alkaline pH between 8 and 9, preferably of between 8.4 and 8.6. Casein is dissolved in water.

In stage 2) N-acetyl-L-aspartyl chloride is prepared *in situ* by N-acetyl-L-aspartic acid and a chlorinating reagent such as thionyl chloride.

In stage 3) N-acetyl-aspartylation is performed by reaction of the dissolved casein of stage 1) with N-acetyl-L-aspartyl chloride prepared in stage 2). The reaction can be performed at a temperature in the range of from 15°C to 40°C, such as at room temperature (20°C), preferably it is performed at a temperature in a range of from 30 to 40°C. Preferably, the pH-value is kept within the range of pH 8-9 during reaction of casein with N-acetyl-L-aspartyl chloride.

The N-acetyl-L-aspartyl chloride is prepared in situ (without any isolation) and set for reaction with soluble casein. The N-acetyl-L-aspartic acid ratio to the casein, which is preferably used, is 1:2.

In stage 4) the N-acetyl-L-aspartylated product is purified after precipitation via acidification at a pH value of between 2.5 and 4, preferably of between 2.5 and 3, and separated by filtration.

In stage 5), the product is recovered by filtration, is suspended in water and sodium hydroxide and subjected to dilaceration at a pH of between 6 and 11, preferable 8 and 9, until a solution is obtained.

### Step (b):

In stage 6) the reaction with ferric chloride may be performed by adding an aqueous solution of ferric chloride to the solution obtained at the end of stage 5), until a pH of between 2.5 and 3 is obtained. A suspension is formed, which is subjected to filtration, and crude ferro-N-acetyl-L-aspartylated casein is recovered as insoluble phase.

5 g of modified casein can be reacted with 6.2 g FeCl₃ 26.9 % w/w solution.

In stage 7), the purification is performed as follows: the crude derivative obtained in stage 6) is suspended in water, alkalized at pH 8.5 and then agitated for 30 minutes until the product dissolution. The purified complex is clarified by subsequent filtration of the solution.

In stage 8) the ferro-N-acetyl-L-aspartylated casein is precipitated from the clarified solution of stage 7) by acidification at pH 2.5 and is then filtered and recovered.

In stage 9), the ferro-N-acetyl-L-aspartylated casein complex undergoes desiccation at a temperature of 60 °C and 80 °C), for a period of time of between 18 and 36 hours, preferably between 20 and 24 hours.

At the end of desiccation, the product contains a maximum moisture residue of less than 5% and a content of complexed iron of between 5% and 6% by weight, calculated on the dry substance. For a complete characterization of the product, reference is made to the data provided in Examples 1 to 3.

The process described makes it possible to obtain ferro-N-acetyl-L-aspartylated casein complex free from residue or from iron derivatives that are insoluble or poorly soluble in water. In particular, the product is found to be completely soluble at alkaline pH values, i.e., the ones typical of the intestinal tract, thus guaranteeing maximum iron bio-availability for the purpose of intestinal absorption.

The product according to the present invention can be adequately compounded in pharmaceutical formulations suitable for oral administration.

The present invention therefore comprises pharmaceutical compositions containing ferro-N-acetyl-L-aspartylated casein and appropriate excipients, such as diluents and pharmaceutical preservatives.

A further subject of the invention refers to the use of the ferric complexes described above for the preparation of medicaments for the treatment of pathological conditions resulting from iron deficiency due, for example, to physiological alteration, periodic hemorrhage, infective diseases, pregnancy, breast-feeding, and imperfect metabolic utilization. One example of these pathological conditions is anaemia, in particular hypochromic sideropenic anaemia.

In order to provide an illustration of the present invention, which in no way limits the scope thereof, the following experimental examples are given.

### EXAMPLES

### Example 1- Preparation of Iron Casein N-acetyl-L-aspartylated Complex

### (A) Casein dissolution.

In a stainless steel reactor with sufficient shaker and pH detector, are added 5 g of casein used for food or pharmaceutical purposes on 70 g of de-ionized, microbiologically pure water. The mixture is kept under stirring until a homogeneous suspension is obtained. Approximately 1.5 g of a 15 % w/w sodium hydroxide (NaOH) solution is added, at a temperature of 20 °C, in a period of 10 minutes, until a pH value of 8.5 ± 0.1 is obtained. The solution is shaken for another 15 minutes.

### (B) N-Acetyl-L-Aspartyl Chloride preparation

In a stainless steel reactor with sufficient shaker are added, 15.6 mL (15 equivalents) of thionyl chloride (SOCl₂), 2.5 g of N-acetyl-L-aspartic acid and the mixture is kept under stirring at 75 °C. The mixture is kept under stirring until the reaction completion (1-1,5 h) by the formation of the acyl chloride product. In this reaction, the sulfur dioxide (SO₂) and hydrogen chloride (HCl) generated are both gases that can leave the reaction vessel, driving the reaction forward. The excess of thionyl chloride is evaporated by heating the reaction mixture at 75 °C, close to the boiling point (74.6 °C) of the SOCl₂.

### (C) Casein acetylaspartylation.

The solution obtained from stage (A), of the dissolved casein, is added gradually to the, the N-acetyl-L-aspartic chloride (B), with simultaneously addition about 3.1 g of 15 % w/w NaOH, maintaining pH at 8.5 ± 0.1, at room temperature. The solution obtained is left under strong agitation for 2h.

### (D) Transfer and purification of casein acetyl aspartate via precipitation.

The solution obtained from stage (C) is transferred into an enameled reactor provided with a mixer and a suitable probe for reading pH values. The solution, kept at a temperature of 20 °C, is then acidified with approximately 2.28 g of HCl 6 N solution in a period of 40 minutes, until a pH of 3 is obtained. On reaching the target pH, addition of acid is stopped and precipitate is obtained. The decrease in the pH value causes the formation of a precipitate of N-acetyl-L-aspartylated casein depurated from other salts, other reaction products, and other soluble impurities.

### (E) Filtration and dilacerations.

The N-acetyl-L-aspartylated casein obtained in stage (D) is recovered by filtration. The moist product is removed from the filter, and then put into a stainless-steel reactor equipped with an anchor mixer and with a probe for reading pH values which has been charged with 70 ml of depurated water, and then subjected to agitation. The suspension is kept under agitation for 10 min, at a temperature of 20 °C, and then subjected to dilaceration. After 20 minutes of dilaceration, approximately 3.1 g of NaOH 15 % w/w solution are added to the suspension, until a stable pH value of between 8.1 and 8.4 is obtained. The aspartylated casein suspension is filtered, the reactor and filter are washed with 50 ml of depurated water, and the washing water is then added again to the clarified solution.

### (F). Reaction with ferric chloride and fltration of crude ferro-N-acetyl-L-aspartylated casein.

The solution from stage (E) is sent on to an enamelled reactor equipped with impeller mixer and with a probe for reading pH values. To this homogeneous solution is added 6.2 g in total of FeCl₃.6H₂O 26.9%; w/w (Iron solution), accordingly:
i) Addition of 1.7 g of iron solution with simultaneous incremental addition of 0.6 g 15 % w/w NaOH in order to set the pH value between 8.3-8.5. The solution is left for agitation for 15 minutes.
ii) Addition of the rest 4.5 g of iron solution until the pH of the solution to maintain between 2.8-3.0. The suspension is kept under agitation for 1 h. The pH is adjusted with HCl 6 N solution or 15% w/w NaOH solution, respectively.

A suspension of ferro-N-acetyl-L-aspartylated casein is obtained. The reaction product is kept under agitation for 20 minutes, and the crude ferro-N-acetyl-L-aspartylated casein derivative is recovered by filtration.

### (G) Complex dissolving and filtration.

The solid complex is dispersed in 70 g of purified water and then 3.85 g of NaOH 15 % w/w solution are added until a pH of 8.5 ± 0.1. The solution is kept under agitation for 30 minutes. The remained solution is filtered in order the undissolved impurities to be separated. The insoluble residue is less than 0.5 wt%.

### (H) Precipitation and isolation of pure moist ferro-N-acetyl-L-aspartylated casein.

To the clear solution, which is put into an enamelled reactor equipped with an impeller mixer and with a probe for reading the pH value, are added by pouring 2.28 g of 6 N HCl aqueous solution, at a temperature of between 20 °C and 25 °C, in approximately 20-30 minutes, until a pH value of 2.8-3.0 is obtained. At the end of the addition, the reaction product is kept stirred for 20 minutes, and the precipitate of pure ferro-N-acetyl-L-aspartylated casein that has formed is recovered by filtration and subsequent washing with purified water.

### (I) Desiccation of ferro-N-acetyl-L-aspartylated casein.

The moist pure product obtained in the previous stage is dried under vacuum, at a temperature increasing from 60 °C to 70 °C, for an overall period of 24 hours. After the desiccation process, 6.5 g of pure iron (III) casein N-acetyl-L-aspartylated complex are obtained, with a water content < 5 wt % (Karl Fisher), having the following chemical and physicochemical characteristics:

| **Product characteristics** | **Value** |
|---|---|
| Colour | Brown-red |
| Solubility | soluble in NaOH pH = 8 |
| pH | 2.8 |
| Free iron (not complexed) | <0.05% w/w |
| Total iron | 5.5 % w/w |
| Proteins | 75.2 % w/w |
| Complexed N-acetyl-L-aspartic acid | 7.1 % w/w |
| Free N-acetyl-L-aspartic acid | 0.1 % w/w |
| Chlorides | 1.8 % w/w |
| Bacterial content | <10³ CFU/g |
| Mold and yeast content | < 10² CFU/g |

### Example 2 - Preparation of Iron Casein N-acetyl-L-aspartylated Complex

### (A) Casein dissolution.

In a stainless steel reactor with sufficient shaker and pH detector, are added 5 g of casein used for food or pharmaceutical purposes on 70 g of de-ionized, microbiologically pure water. The mixture is kept under stirring until a homogeneous suspension is obtained. Approximately 1.5 g of a 15 % w/w sodium hydroxide (NaOH) solution is added, at a temperature of 20 °C, in a period of 10 minutes, until a pH value of 8.5 ± 0.1 is obtained. The solution is shaken for another 15 minutes.

### (B) N-Acetyl-L-Aspartyl Chloride preparation

In a stainless steel reactor with sufficient shaker are added, 15.2 mL (15 equivalents) of thionyl chloride (SOCl₂), a catalytic amount of 40 µL of dimethyl formamide (DMF), 2.5 g of N-acetyl-L-aspartic acid and the mixture is heated to 75 °C under reflux conditions. The mixture is kept under stirring until the reaction completion by the formation of the acyl chloride product. In this reaction, the sulfur dioxide (SO₂) and hydrogen chloride (HCl) generated are both gases that can leave the reaction vessel, driving the reaction forward. The excess of thionyl chloride is evaporated by heating the reaction mixture gradually up to 75 °C, close to the boiling point (74.6 °C) of the SOCl₂.

### (C) Casein acetylaspartylation.

The solution obtained from stage (A), of the dissolved casein, is added to the N-acetyl-L-aspartic chloride of stage (B), with simultaneously addition about 3.1 g of 15 % w/w NaOH, maintaining pH at 8.5 ± 0.1, at room temperature. The solution obtained is left under strong agitation for 2h.

### (D) Transfer and purification of casein acetyl aspartate via precipitation.

The solution obtained from stage (C) is transferred into an enameled reactor provided with a mixer and a suitable probe for reading pH values. The solution, kept at a temperature of 20 °C, is then acidified with approximately 2.28 g of HCl 6 N solution in a period of 40 minutes, until a pH of 3 is obtained. On reaching the target pH, addition of acid is stopped and precipitate is obtained. The decrease in the pH value causes the formation of a precipitate of N-acetyl-L-aspartylated casein depurated from other salts, other reaction products, and other soluble impurities.

### (E) Filtration and dilacerations.

The N-acetyl-L-aspartylated casein obtained in stage (D) is recovered by filtration. The moist product is removed from the filter, and then put into a stainless-steel reactor equipped with an anchor mixer and with a probe for reading pH values which has been charged with 70 ml of depurated water, and then subjected to agitation. The suspension is kept under agitation for 1 hour, at a temperature of 20 °C, and then subjected to dilaceration. After 20 minutes of dilaceration, approximately 3.1 g of NaOH 15 % w/w solution are added to the suspension, until a stable pH value of between 8.1 and 8.4 is obtained. At the end of the addition, the reaction product is once more treated in the same reactor, for a period of approximately 2 hours. This operation facilitates the disgregation of the solid particles of N-acetyl-L-aspartylated casein. The aspartylated casein suspension is filtered, the reactor and filter are washed with 50 ml of depurated water, and the washing water is then added again to the clarified solution.

### (F). Reaction with ferric chloride and filtration of crude ferro-N-acetyl-L-aspartylated casein.

The solution from stage (E) is sent on to an enamelled reactor equipped with impeller mixer and with a probe for reading pH values. To this homogeneous solution is added 6.2 g in total of FeCl₃·6H₂O 26.9% w/w (iron solution), accordingly:
i) Addition of 1.7 g of Iron solution with simultaneous incremental addition of 0.6 g 15 % w/w NaOH in order to maintain pH between 8.3-8.5. The solution is left for agitation for 15 minutes.
ii) Addition of the rest 4.5 g of Iron solution until the pH of the solution is between 2.8-3.0. The suspension is kept under agitation for 1 h. The pH is adjusted with HCl 6 N solution or 15% w/w NaOH solution, respectively.

A suspension of ferro-N-acetyl-L-aspartylated casein is obtained. The reaction product is kept under agitation for 20 minutes, and the crude ferro-N-acetyl-L-aspartylated casein derivative is recovered by filtration.

### (G) Complex dissolving and filtration.

The solid complex is dispersed in 70 g of purified water and then 3.85 g of NaOH 15 % w/w solution are added until a pH of 8.5 ± 0.1. The solution is kept under agitation for 30 minutes. The remained solution is filtered in order the undissolved impurities to be separated. The insoluble residue is less than 0.5 wt%.

### (H) Precipitation and isolation of pure moist ferro-N-acetyl-L-aspartylated casein.

To the clear solution, which is put into an enamelled reactor equipped with an impeller mixer and with a probe for reading the pH value, are added by pouring 2.28 g of 6 N HCl aqueous solution, at a temperature of between 20 °C and 25 °C, in approximately 20-30 minutes, until a pH value of 2.8-3.0 is obtained. At the end of the addition, the reaction product is kept stirred for 20 minutes, and the precipitate of pure ferro-N-acetyl-L-aspartylated casein that has formed is recovered by filtration and subsequent washing with purified water.

### (I) Desiccation of ferro-N-acetyl-L-aspartylated casein.

The moist pure product obtained in the previous stage is dried under vacuum, at a temperature increasing from 60 °C to 70 °C, for an overall period of 24 hours. After the desiccation process, 6.6 g of pure iron (III) casein N-acetyl-L-aspartylated complex are obtained, with a water content < 5 wt% (Karl Fisher), having the following chemical and physicochemical characteristics:

| **Product characteristics** | **Value** |
|---|---|
| Colour | Brown-red |
| Solubility | Soluble in NaOH pH = 8 |
| pH | 2.7 |
| Free Iron (not complexed) | <0.05% w/w |
| Total iron | 5.7 % w/w |
| Proteins | 84.1 % w/w |
| Complexed N-acetyl-L-aspartic acid | 7.3 % w/w |
| Free N-acetyl-L-aspartic acid | 0.2 % w/w |
| Chlorides | 1.6 % w/w |
| Bacterial content | <10³ CFU/g |
| Mold and yeast content | <10² CFU/g |

### Example 3 - Preparation of Iron Casein N-acetyl-L-aspartylated Complex

### (A) Casein dissolution.

In a stainless steel reactor with sufficient shaker and pH detector, are added 5 g of casein used for food or pharmaceutical purposes on 70 g of de-ionized, microbiologically pure water. The mixture is kept under stirring until a homogeneous suspension is obtained. Approximately 1.5 g of a 15 % w/w sodium hydroxide (NaOH) solution is added, at a temperature of 20 °C, in a period of 10 minutes, until a pH value of 8.5 ± 0.1 is obtained. The solution is shaken for another 15 minutes.

### (B) N-Acetyl-L-Aspartyl Chloride preparation

In a stainless steel reactor with sufficient shaker are added, 15.6 ml (15 equivalents) of thionyl chloride (SOCl₂), a catalytic amount of 100 µL of dimethylformamide (DMF) and 2.5 g of N-acetyl-L-aspartic acid. The mixture is kept under stirring at 75 °C under reflux conditions until the reaction completion by the formation of the acyl chloride product. In this reaction, the sulfur dioxide (SO₂) and hydrogen chloride (HCl) generated are both gases that can leave the reaction vessel, driving the reaction forward. The excess of thionyl chloride is evaporated by heating the reaction mixture gradually up to 75 °C, without the cooling condenser, close to the boiling point (74.6 °C) of the SOCl₂.

### (C) Casein acetylaspartylation.

The solution obtained from stage (A), of the dissolved casein, is added to the N-acetyl-L-aspartic chloride of stage (B), with simultaneously addition about 3.1 g of 15 % w/w NaOH, maintaining pH at 8.5 ± 0.1, at room temperature. The solution obtained is left under strong agitation for 2h.

### (D) Transfer and purification of casein acetyl aspartate via precipitation.

The solution obtained from stage (C) is transferred into an enameled reactor provided with a mixer and a suitable probe for reading pH values. The solution, kept at a temperature of 20 °C, is then acidified with approximately 2.28 g of HCl 6 N solution in a period of 40 minutes, until a pH of 3 is obtained. On reaching the target pH, addition of acid is stopped and precipitate is obtained. The decrease in the pH value causes the formation of a precipitate of N-acetyl-L-aspartylated casein depurated from other salts, other reaction products, and other soluble impurities.

### (E) Filtration and dilacerations.

The N-acetyl-L-aspartylated casein obtained in stage (D) is recovered by filtration. The moist product is removed from the filter, and then put into a stainless-steel reactor equipped with an anchor mixer and with a probe for reading pH values which has been charged with 70 ml of depurated water, and then subjected to agitation. The suspension is kept under agitation for 1 hour, at a temperature of 20 °C, and then subjected to dilaceration. After 20 minutes of dilaceration, approximately 3.1 g of NaOH 15 % w/w solution are added to the suspension, until a stable pH value of between 8.1 and 8.4 is obtained. At the end of the addition, the reaction product is once more treated in the same reactor, for a period of approximately 2 hours. This operation facilitates the dilaceration and then solubilization of the solid particles of N-acetyl-L-aspartylated casein. The aspartylated casein suspension is filtered, the reactor and filter are washed with 50 ml of depurated water, and the washing water is then added again to the clarified solution.

### (F). Reaction with ferric chloride and filtration of crude ferro-N-acetyl-L-aspartylated casein.

The solution from stage (E) is sent on to an enamelled reactor equipped with impeller mixer and with a probe for reading pH values. To this homogeneous solution is added 6.2 g in total of FeCl₃·6H₂O 26.9% w/w (Iron solution), accordingly:
i) Addition of 1.7 g of iron Solution with simultaneous incremental addition of 0.6 g 15 % w/w NaOH in order to set the pH at between 8.3-8.5. The solution is left for agitation for 15 minutes.
ii) Addition of the rest 4.5 g of Iron solution until the pH of the solution is between 2.8-3.0. The suspension is kept under agitation for 1 h. The pH is adjusted with HCl 6 N solution or 15% w/w NaOH solution, respectively.

A suspension of ferro-N-acetyl-L-aspartylated casein is obtained. The reaction product is kept under agitation for 20 minutes, and the crude ferro-N-acetyl-L-aspartylated casein derivative is recovered by filtration.

### (G) Complex dissolving and filtration.

The solid complex is dispersed in 70 g of purified water and then 3.85 g of NaOH 15 % w/w solution are added until a pH of 8.5 ± 0.1. The solution is kept under agitation for 30 minutes. The retained solution is filtered in order the undissolved impurities to be separated. The insoluble residue is less than 0.5 wt%.

### (H) Precipitation and isolation of pure moist ferro-N-acetyl-L-aspartylated casein.

To the clear solution, which is put into an enamelled reactor equipped with an impeller mixer and with a probe for reading the pH value, are added by pouring 2.28 g of 6 N HCl aqueous solution, at a temperature of between 20 °C and 25 °C, in approximately 20-30 minutes, until a pH value of 2.8-3.0 is obtained. At the end of the addition, the reaction product is kept stirred for 20 minutes, and the precipitate of pure ferro-N-acetyl-L-aspartylated casein that has formed is recovered by filtration and subsequent washing with purified water.

### (I) Desiccation of ferro-N-acetyl-L-aspartylated casein.

The moist pure product obtained in the previous stage is dried under vacuum, at a temperature increasing from 60 °C to 70 °C, for an overall period of 24 hours. After the desiccation process, 6.4 g of pure iron (III) casein N-acety)-L-aspartyiated complex are obtained, with a water content < 5 wt% (Karl Fisher), having the following chemical and physicochemical characteristics:

| **Product characteristics** | **Value** |
|---|---|
| Colour | Brown-red |
| Solubility | Soluble in NaOH pH = 8 |
| pH | 2.8 |
| Free iron (not complexed) | <0.05% w/w |
| Total iron | 5.3 % w/w |
| Proteins | 88.0 % w/w |
| Complexed N-acetyl-L-aspartic acid | 7.0 % w/w |
| Free N-acetyl-L-aspartic acid | 0.6 % w/w |
| Chlorides | 1.8 % w/w |
| Bacterial content | < 10³ CFU/g |
| Mold and yeast content | < 10² CFU/g |

### Cited literature

1. GB910321 "Improvements in and relating to a process of preparing Ferrous Salts of Aspartic Acid and Medicines containing these Salts".
2. Arzneimittel-Forschung, 1984; 34(9):948-952 "Iron derivatives of modified milk protein".
3. US4493829, 1985, "Bio-available succinylated protein-iron derivatives which do not cause gastric lesions, method of preparation and related pharmaceutical compounds".
4. EP0243322, 1987, "Compounds containing bioavailable iron, process for their preparation and pharmaceutical compositions containing them".
5. International Journal of Clinical Pharmacology, therapy, and toxicology, 1993; 31(1):40-51 "Chemical and biological characterization of iron-protein succinylate (ITF 282)".
6. EP0939083 "Ferro-succinylcasein complex, process for its preparation and pharmaceutical compositions containing it".
7. US2001031748 "Use of metal complexes to treat gastrointestinal infections".
8. Clinical Drug Investigation, 2005; 25(11):679-689 "Overview of clinical trials in the treatment of iron deficiency with iron-acetyl-aspartylated casein".
9. WO2006021843 "A process for producing iron succinyl casein and acetyl-aspartate iron casein complexes and use thereof in pharmaceutical mixtures".
10. Tetrahedron: Asymmetry 18 (2007) 1625-1627.

## Claims

1. Process for the preparation of iron (III) complex with N-acetyl-L-aspartylated casein, comprising the steps of
(a) reaction of casein with N-acetyl-L-aspartyl chloride, to form N-acetyl-L-aspartylated casein,
(b) subsequent reaction of the N-acetyl-L-aspartylated casein with ferric chloride; and
(c) obtaining the iron (III) complex with N-acetyl-L-aspartylated casein.

2. The process of claim 1, wherein the casein utilized is food-grade casein.

3. The process of claim 1, wherein step (a) comprises providing a solution of casein, and wherein addition of N-acetyl-L-aspartyl chloride to the casein solution is performed at a pH of between 6 and 9.

4. The process of claim 1, wherein the N-acetyl-L-aspartyl chloride is prepared *in situ,* preferably by using chlorinating reagents selected from the group consisting of thionyl chloride (SOCl₂), phosphorus pentachloride (PCl₅), phosphorus trichloride (PCl₃), carbon tetrachloride (CCl₄), oxalyl chloride (COCl)₂, phosphoryl chloride (POCl₃), 2-chloro-1,3-bis(methoxycarbonyl)guanidine (Palau'Chlor), cyanuric chloride and *N*-chlorosuccinimide.

5. The process of claim 1, wherein the iron (III) complex with N-acetyl-L-aspartylated casein obtained in step (c) is clarified before being sent on for desiccation or spray-drying or freeze-drying.

6. A process for preparing a pharmaceutical composition comprising an iron (III) complex with N-acetyl-L-aspartylated food-grade casein, said process comprising the process according to anyone of claims 1-5 and combining said iron (III) complex with N-acetyl-L-aspartylated food-grade casein with pharmaceutical excipients, in particular selected from diluents and preservatives.

## Patentansprüche

1. Verfahren zur Herstellung eines Eisen (III)-Komplexes mit N-Acetyl-L-aspartyliertem Casein, umfassend die Schritte
(a) Reaktion von Casein mit N-Acetyl-L-Aspartylchlorid, um N-Acetyl-L-aspartyliertes Casein zu bilden,
(b) anschließende Reaktion des N-Acetyl-L-aspartyliertem Caseins mit Eisenchlorid; und
(c) Erhalten des Eisen (III)-Komplexes mit N-Acetyl-L-aspartyliertem Casein.

2. Verfahren nach Anspruch 1, worin das eingesetzte Casein Casein vom Nahrungsmittelgrad ist.

3. Verfahren nach Anspruch 1, worin der Schritt (a) das Bereiten einer Lösung des Caseins umfasst, und worin die Zugabe des N-Acetyl-L-Aspartylchlorids zu der Casein-Lösung bei einem pH von zwischen 6 und 9 durchgeführt wird.

4. Verfahren nach Anspruch 1, worin das N-Acetyl-L-Aspartylchlorid *in situ* hergestellt wird, vorzugsweise unter Verwendung von Chlorierungsreagenzien, ausgewählt aus der Gruppe bestehend aus Thionylchlorid (SOCl₂), Phosphorpentachlorid (PCl₅), Phosphortrichlorid (PCl₃), Kohlenstofftetrachlorid (CCl₄), Oxalylchlorid (COCl)₂, Phosphorylchlorid (POCl₃), 2-Chloro-1,3-bis(methoxycarbonyl)guanidin (Palau 'Chlor), Cyanurchlorid und N-Chlorsuccinimid.

5. Verfahren nach Anspruch 1, worin der Eisen (III)-Komplex mit N-Acetyl-L-aspartyliertem Casein, erhalten im Schritt (c), geklärt wird, bevor er zur Entwässerung oder zum Sprühtrocknen oder Gefriertrocknen weitergeleitet wird.

6. Verfahren zur Herstellung einer pharmazeutischen Zusammensetzung, umfassend einen Eisen (III)-Komplex mit N-Acetyl-L-aspartyliertem Casein vom Nahrungsmittelgrad, wobei das Verfahren das Verfahren gemäß irgendeinem der Ansprüche 1-5 umfasst, und die Kombinierung des Eisen (III)-Komplexes mit N-Acetyl-L-aspartyliertem Casein vom Nahrungsmittelgrad mit pharmazeutischen Hilfsstoffen, insbesondere ausgewählt aus Verdünnungsmitteln und Konservierungsstoffen.

## Revendications

1. Procédé de préparation d'un complexe de fer (III) avec une caséine N-acétyl-L-aspartylée, comprenant les étapes de :
(a) réaction de la caséine avec du chlorure de N-acétyl-L-aspartyle, pour former de la caséine N-acétyl-L-aspartylée,
(b) réaction subséquente de la caséine N-acétyl-L-aspartylée avec du chlorure ferrique ; et
(c) obtention du complexe de fer (III) avec la caséine N-acétyl-L-aspartylée.

2. Procédé selon la revendication 1, dans lequel la caséine utilisée est une caséine de qualité alimentaire.

3. Procédé selon la revendication 1, dans lequel l'étape (a) comprend la fourniture d'une solution de caséine, et dans lequel l'ajout de chlorure de N-acétyl-L-aspartyle à la solution de caséine est effectué à un pH compris entre 6 et 9.

4. Procédé selon la revendication 1, dans lequel le chlorure de N-acétyl-L-aspartyle est préparé *in situ,* de préférence en utilisant des réactifs de chloration choisis dans le groupe constitué par le chlorure de thionyle (SOCl₂), le pentachlorure de phosphore (PCl₅), le trichlorure de phosphore (PCl₃), le tétrachlorure de carbone (CCl₄), le chlorure d'oxalyle (COCl)₂, le chlorure de phosphoryle (POCl₃), la 2-chloro-1,3-bis(méthoxycarbonyl)guanidine (Palau'Chlor), le chlorure de cyanure et le *N-*chlorosuccinimide.

5. Procédé selon la revendication 1, dans lequel le complexe de fer (III) avec la caséine N-acétyl-L-aspartylée obtenu à l'étape (c) est clarifié avant d'être envoyé pour la dessiccation ou le séchage par pulvérisation ou la lyophilisation.

6. Procédé de préparation d'une composition pharmaceutique comprenant un complexe de fer (III) avec une caséine N-acétyl-L-aspartylée de qualité alimentaire, ledit procédé comprenant le procédé selon l'une quelconque des revendications 1 à 5 et la combinaison dudit complexe de fer (III) avec la caséine N-acétyl-L-aspartylée de qualité alimentaire avec des excipients pharmaceutiques, choisis en particulier parmi les diluants et les conservateurs.
